# EUROPEAN PATENT SPECIFICATION

(11) **EP 0 822 781 B1**
(45) Date of publication and mention of the grant of the patent: **05.07.2000**
(21) Application number: 96911056.8
(22) Date of filing: 25.04.1996
(51) Int. Cl.: A61B 17/04, A61B 17/28

(54) **SURGICAL GRIPPING DEVICE**
CHIRURGISCHE GREIFVORRICHTUNG
PINCE CHIRURGICALE

(30) Priority: 25.04.1995 GB 9508328
(43) Date of publication of application: 11.02.1998
(73) Proprietor: THE UNIVERSITY OF NOTTINGHAM, Nottingham NG7 2RD (GB); UNIVERSITY OF DUNDEE, Dundee DD1 4HN (GB)
(72) Inventor: WOOD, John, Vivian, Nottingham NG7 2RD (GB); GRANT, David, Malcolm, Nottingham NG7 2RD (GB); GREEN, Sarah, Margaret, Nottingham NG7 2RD (GB); BENHAM, Timothy, 81 Gothenburg (SE); GARLICK, David, Nottingham NG7 2RD (GB)
(74) Representative: Lock, Howard John
(86) International application number: GB9600998
(87) International publication number: WO9633660

(56) References cited:
- WO-A-95/11629
- DE-C- 759 986
- US-A- 4 839 479
- US-A- 5 242 458

## Description

The present invention concerns a gripping device for surgical use, and relates in particular although not exclusively to a biocompatible surgical needle holder having deformable gripping surfaces.

Surgical needle holders, generally in the nature of forceps, are known and used in both general and laparoscopic surgery. Conventionally, the gripping surface or surfaces of such holders are machined to a knurled finish or coated with a relatively hard and rough coating, for example tungsten carbide, to stop the needle from moving or from dropping out of the needle holder during an operation. Gripping surfaces of this nature tend to distort the needle and may remove a surface coating from the needle as well as removing part of the needle material itself, which is commonly a type of stainless steel. This distortion and degradation of the needle causes problems, particularly in accurate surgical work. Moreover, it is known occasionally for the needle to be pulled out of the holder during surgery, or to twist within the holder resulting in incorrect alignment of a curved needle.

Specification No. US-A-5 242 458 discloses a suture needle holder for endoscopic use.

The present invention seeks to mitigate or obviate these or other disadvantages of the prior art.

According to the invention there is provided a biocompatible gripping device (10) for surgical use including at least one deformable gripping surface (20), the surface comprising a shape memory material, characterized in that the shape memory material comprises residual porosity.

The surface is deformable to conform at least in part to the shape of an object gripped thereby to thereby provide enhanced grip of the object. The surface comprises a shape memory material, and in particular a shape memory alloy which may return to a non-deformed condition through a shape memory phase transformation upon heating.

The term shape memory material is used herein to refer to a material which recovers from a deformed shape to a pre-formed, substantially stress-free shape on being subjected to certain predetermined conditions.

Preferably the device comprises a pair of cooperating gripping members, each of which provides a gripping surface whereby an article may be held between the surfaces.

Preferably a coating or an insert of the shape memory alloy is provided on each gripping member to form the respective gripping surface.

The shape memory alloy may be a titanium-nickel alloy, preferably a nominally equiatomic alloy, with a composition of desirably between 48 and 52 atomic % nickel.

The alloy coating or insert may be applied or attached by brazing, soldering, riveting, sintering or compression fit.

Preferably the device is a surgical needle holder, desirably in the form of forceps.

The invention will be further described for the purposes of illustration only with reference to the following accompanying drawings in which:-
Fig. 1 is a diagrammatic drawing of a surgical needle holder made in accordance with the invention; and
Fig. 2 shows schematically the operation of the jaw inserts.

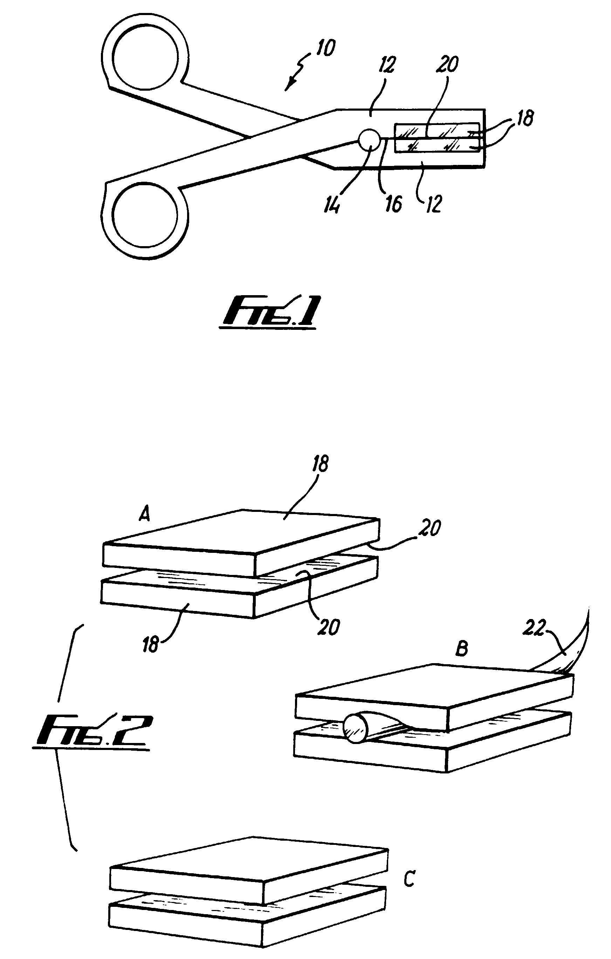

Referring to Fig. 1, a stainless steel surgical needle holder 10 takes the general form of a pair of forceps. The holder 10 has a pair of jaws 12 movable about a pivot 14. Each jaw 12 has an inner surface 16 in which is provided an insert 18. Each insert 18 provides a gripping surface 20 so that the respective gripping surfaces 20 come into contact with one another when the needle holder is in a closed condition.

Each insert 18 is made from a nominally equiatomic nickel-titanium shape memory alloy, and is formed by a process which will be described hereinafter. The gripping surface 20 provided by the alloy insert 18 is deformable on the application of a force such as may be applied to hold a conventional stainless steel surgical needle 22 in place within the jaws 12 of the needle holder (see Fig. 2B). The inserts 18 thus deform when a needle is gripped enabling a secure and accurate grip to be achieved without damage to the needle itself. The nickel-titanium shape memory alloy has a relatively high coefficient of friction and effectively acts as a sticky material gripping the needle.

The size of the inserts permits several gripping operations to be made bfore the insert is substantially deformed over its surface. The insert material then requires to be subjected to appropriate conditions to cause shape recovery, to return it to its original (undeformed) condition. The nickel-titanium alloy employed in the present example has a martensite to austenite phase transformation temperature occurring between 50°C and 100°C, and its shape memory effect can therefore be realised either by immersion in hot water or by routine autoclave sterilisation.

The inserts 18 are produced from elemental pure nickel and titanium powders. The powders are mixed in the approximate ratio 50 at % Ni-Ti, cold compacted and subjected to an inert atmosphere (argon) sinter. The resulting sintered compact contains closed porosity, the extent of which can be controlled by variation of the cold compaction pressure and the initial particle size of the nickel and titanium powders. Modification of the theoretical density of the jaw inserts can thus be achieved. The powder process Ni-Ti intermetallic exhibits the shape memory effect, with a martensite to austenite phase transformation temperature occurring between 50 and 100°C, dependent upon the composition.

The inserts 18 may be attached to the needle holder either by rivetting, soldering, sintering or brazing. The present example employs a type of silver solder, namely a silver-copper-zinc-tin alloy supplied by Eutectic Co. Ltd. of Worcestershire, under the name Superflux 1020. The corresponding flux permits the solder to wet the stainless steel of the needle holder 10 relatively easily. To coat the alloy insert, it was first covered in molten flux, then a small quantity of the solder was melted on it. Oxide forming on the surface was scratched through the solder with an appropriate pointed stainless steel instrument. With the alloy insert held at a suitable degree of super-heat, the solder flowed under the oxide film thus lifting it off. The slag was scraped off and fresh flux applied as protection.

When both surfaces had been coated with solder, they werre joined and re-heated until they sweated, ensuring that the correct relative positions were retained.

There is thus provided a surgical needle holder which enables a good grip to be obtained without significant likelihood of damage to the needle.

Modifications may be made within the scope of the invention. In particular, a deformable gripping surface may be provided by other materials than those described, and may be provided on the needle holder in any convenient manner. The invention extends to surgical equipment other than needle holders.

Whilst endeavouring in the foregoing Specification to draw attention to those features of the invention believed to be of particular importance it should be understood that the protection claimed by the Applicant is only limited by the appended claims.

## Claims

1. A biocompatible gripping device (10) for surgical use including at least one deformable gripping surface (20), the surface comprising a shape memory material, characterized in that the shape memory material comprises residual porosity.

2. A device (10) as claimed in claim 1, in which the surface (20) comprises a shape memory alloy.

3. A device (10) as claimed in claim 2 in which the alloy is capable of removing deformation by shape memory phase transformation upon heating.

4. A device (10) as claimed in any preceding claim, in which the device (10) comprises a pair of cooperating gripping members (12), each of which provides a gripping surface (20) whereby an article (22) may be held between the surfaces (20).

5. A device (10) as claimed in claim 4, in which a coating or an insert (18) of the shape memory material is provided on each gripping member (12) to form the respective gripping surface (20).

6. A device (10) as claimed in any of claims 2 to 5, in which the shape memory alloy is a titanium-nickel alloy.

7. A device (10) as claimed in claim 6 in which the alloy comprises between 48 and 52 atomic % nickel.

8. A device (10) as claimed in any of claims 4 to 7, in which the shape memory alloy is a nominally equiatomic alloy.

9. A device (10) as claimed in any of claims 3 to 8, in which the alloy coating or insert (18) is applied or attached by brazing, soldering, rivetting, sintering or compression fit.

10. A device (10) as claimed in any preceding claim, in which the device (10) is a surgical needle holder.

11. A device (10) as claimed in any of claims 1 to 9, in which the device (10) is in the form of forceps.

12. The use of a device (10) as claimed in any preceding claim to grip an object (22) wherein the surface (20) is deformable to conform at least in part to the shape of the object (22) gripped thereby to provide enhanced grip of the object (22).

## Patentansprüche

1. Biokompatible Greifvorrichtung (10) zur chirurgischen Verwendung mit mindestens einer verformbaren Greiffläche (20), wobei die Fläche ein Formgedächtnis-Material aufweist, dadurch gekennzeichnet, daß das Formgedächtnis-Material eine Restporosität aufweist.

2. Vorrichtung (10) nach Anspruch 1, dadurch gekennzeichnet, daß die Fläche (20) eine Formgedächtnis-Legierung aufweist.

3. Vorrichtung (10) nach Anspruch 2, dadurch gekennzeichnet, daß die Legierung in der Lage ist, eine Verformung mittels Formgedächtnis-Phasenumwandlung durch Erhitzen zu beseitigen.

4. Vorrichtung (10) nach einem der vorhergehenden Ansprüche, dadurch gekennzeichnet, daß die Vorrichtung (10) ein Paar zusammenwirkender Greifglieder (12) aufweist, von denen jedes eine Greiffläche (20) erzeugt, wobei ein Gegenstand (22) zwischen den Flächen (20) gehalten werden kann.

5. Vorrichtung (10) nach Anspruch 4, dadurch gekennzeichnet, daß eine Beschichtung oder eine Einlage (18) aus dem Formgedächtnis-Material an jedem Greifglied (12) vorgesehen ist, um die jeweilige Greiffläche (20) zu bilden.

6. Vorrichtung (10) nach einem der Ansprüche 2 bis 5, dadurch gekennzeichnet, daß die Formgedächtnis-Legierung eine Titan-Nickel-Legierung ist.

7. Vorrichtung (10) nach Anspruch 6, dadurch gekennzeichnet, daß die Legierung zwischen 48 und 52 Atom-% Nickel aufweist.

8. Vorrichtung (10) nach einem der Ansprüche 4 bis 7, dadurch gekennzeichnet, daß die Formgedächtnis-Legierung eine nominal gleichatomige Legierung ist.

9. Vorrichtung (10) nach einem der Ansprüche 3 bis 8, dadurch gekennzeichnet, daß die Legierungsbeschichtung oder -einlage (18) durch Löten bzw. Hartlöten, Schweißen, Nieten, Sintern oder Aufpressen aufgetragen oder befestigt wird.

10. Vorrichtung (10) nach einem der vorhergehenden Ansprüche, dadurch gekennzeichnet, daß die Vorrichtung (10) ein chirurgischer Nadelhalter ist.

11. Vorrichtung nach einem der Ansprüche 1 bis 9, dadurch gekennzeichnet, daß die Vorrichtung (10) die Gestalt einer Zange hat.

12. Verwendung einer Vorrichtung (10) gemäß einem der vorhergehenden Ansprüche zum Ergreifen eines Gegenstands (22), dadurch gekennzeichnet, daß die Fläche (20) verformbar ist, um sich zumindest teilweise der Gestalt des dabei ergriffenen Gegenstands (22) anzupassen, um einen verbesserten Halt des ergriffenen Gegenstands (22) zu erzeugen.

## Revendications

1. Dispositif de préhension biocompatible (10) à usage chirurgical, comprenant au moins une surface de préhension déformable (20), la surface comprenant un matériau à mémoire de forme, caractérisé en ce que le matériau à mémoire de forme comprend une porosité résiduelle.

2. Dispositif (10) selon la revendication 1, dans lequel la surface (20) comprend un alliage à mémoire de forme.

3. Dispositif (10) selon la revendication 2, dans lequel l'alliage est capable de supprimer une déformation par transformation de la phase à mémoire de forme lors du chauffage.

4. Dispositif (10) selon l'une quelconque des revendications précédentes, dans lequel le dispositif (10) comprend une paire d'éléments de préhension (12) qui coopèrent, chacun d'eux fournissant une surface de préhension (20) permettant à un article (22) d'être retenu entre les surfaces (20).

5. Dispositif (10) selon la revendication 4, dans lequel un revêtement ou une pièce d'insertion (18) du matériau à mémoire de forme est prévu sur chaque élément de préhension (12) pour former la surface de préhension respective (20).

6. Dispositif (10) selon l'une quelconque des revendications 2 à 5, dans lequel l'alliage à mémoire de forme est un alliage de titane et de nickel.

7. Dispositif (10) selon la revendication 6, dans lequel l'alliage comprend entre 48 et 52 % atomique de nickel.

8. Dispositif (10) selon l'une quelconque des revendications 4 à 7, dans lequel l'alliage à mémoire de forme est un alliage nominalement équiatomique.

9. Dispositif (10) selon l'une quelconque des revendications 3 à 8, dans lequel le revêtement ou la pièce d'insertion (18) en alliage est appliqué ou attaché par brasage, soudage, rivetage, frittage ou ajustement par compression.

10. Dispositif (10) selon l'une quelconque des revendications précédentes, dans lequel le dispositif (10) est un porte-aiguille chirurgical.

11. Dispositif (10) selon l'une quelconque des revendications 1 à 9, dans lequel le dispositif (10) se présente sous la forme de pinces.

12. Utilisation d'un dispositif (10) selon l'une quelconque des revendications précédentes, pour saisir un objet (22), dans lequel la surface (20) est déformable pour épouser au moins en partie la forme de l'objet (22) saisi par celui-ci afin de permettre une meilleure préhension de l'objet (22).
